# EUROPÄISCHE PATENTSCHRIFT

(11) **EP 3 212 131 B1**
(45) Veröffentlichungstag und Bekanntmachung des Hinweises auf die Patenterteilung: **06.12.2023**
(21) Anmeldenummer: 15808327.9
(22) Anmeldetag: 14.10.2015
(51) Int. Cl.: A61F 2/64, A61F 2/50, A61F 2/74

(54) **PROTHESE**
PROSTHESIS
PROTHÈSE

(30) Priorität: 27.10.2014 DE 102014015756
(43) Veröffentlichungstag der Anmeldung: 06.09.2017
(73) Patentinhaber: Ottobock SE & Co. KGaA, 37115 Duderstadt (DE)
(72) Erfinder: PUSCH, Martin, 37115 Duderstadt (DE); WILL, Christian, 37077 Göttingen (DE); BOITEN, Herman, 37085 Göttingen (DE)
(74) Vertreter: Gramm, Lins & Partner Patent- und Rechtsanwälte PartGmbB
(86) Internationale Anmeldenummer: PCT/DE2015/100426
(87) Internationale Veröffentlichungsnummer: WO 2016/066157

(56) Entgegenhaltungen:
- EP-A1- 0 713 689
- EP-A1- 2 316 390
- EP-B1- 0 713 689
- EP-B1- 2 316 390
- US-A1- 2012 150 318

## Beschreibung

Die Erfindung betrifft eine Prothese mit einem Trägerteil, an dem ein Schwenkgelenk ausgebildet oder befestigt ist. Die Prothese kann als Ersatz für fehlende obere und untere Extremitäten eingesetzt werden, beispielsweise mit einem Prothesenfuß, einem Prothesenknöchelgelenk und einem Unterschenkelschaft, als eine AKP (above knee prosthesis) mit einem Oberschenkelschaft, einem Prothesenkniegelenk und einer distalen Prothesenkomponente in Gestalt eines Unterschenkelrohrs oder eines Unterschenkelteils mit Anschlusselementen für einen Prothesenfuß oder als Prothese für eine obere Extremität, beispielsweise mit einem Ellenbogengelenk zum gelenkigen Verbinden eines Oberarmschaftes mit einem Unterarmteil, das Aufnahmeeinrichtungen für eine Prothesenhand aufweist. Die Prothese ist auf die genannten Anwendungsgebiete nicht beschränkt, für diese jedoch besonders gut geeignet. Die Prothese ist insbesondere für die Versorgung langer Stümpfe und Exartikulationsstümpfe geeignet, bei denen die Schwenkachse sehr nah an dem Stumpfende oder proximal zu dem Stumpfende angeordnet ist.

Die EP 0 713 689 A1 betrifft ein Prothesenkniegelenk mit einer mehrgliedrigen kinematischen Gelenkkette mit mindestens vier Gelenkgliedern, in der jeweils miteinander verbundene Gelenkglieder eine gemeinsame Drehachse aufweisen, die im Wesentlichen parallel zueinander orientiert sind. In der Ausgangsposition des mehrgliedrigen Gelenksystems ist ein Gelenkglied gegenüber den mit ihm verbundenen anderen Gelenkgliedern zwei Bewegungen ausführen kann, wobei wenigstens eine Bewegung, nach deren Einleitung, die andere mögliche Bewegung zumindest überwiegend sperrt. Zumindest ein Gelenkglied kann federbelastet sein.

Die EP 2 316 390 A1 betrifft ein Kniegelenk für eine Prothese, umfassend eine Schwungphasensteuerung mit einem mit einem Oberschenkelverbindungsstück gekoppelten, gegen einen Zylinder bewegbaren Kolben und dem mit einem Oberschenkelverbindungsstück gekoppelten Zylinder, wobei der Zylinder gegen das Unterschenkelverbindungsstück um wenigstens eine Achse verschwenkbar ist. Der Zylinder ist über einen achsfernen Anlenkpunkt an ein sich an dem Unterschenkelverbindungsstück abstützendes Federmittel gekoppelt.

Die US 2012/0150318 A1 wird als nächstliegender Stand der Technik angesehen und betrifft eine Prothese für untere Extremitäten mit einem Aufnahmeelement zur Aufnahme eines Oberschenkelschaftes, einem Trägerteil mit einem Schwenkgelenk sowie einer distalen Prothesenkomponente, die über das Schwenkgelenk gelenkig an dem Trägerteil befestigt ist. Trägerteil und distale Prothesenkomponente bilden das Oberteil und Unterteil eines Prothesenkniegelenkes. An dem Trägerteil ist über einen Ausleger ein Lenker in posteriorer Anordnung befestigt, dessen distales Ende an einem zweiarmigen Schwenkhebel angelenkt ist. Der Schwenkhebel wird bei Flexion und Extension des Prothesenkniegelenkes um eine Schwenkachse verschwenkt, die an dem Unterschenkelteil festgelegt ist. Auf der der Schwenkachse abgewandten Seite des Hebels ist eine Dämpfereinrichtung angeordnet, die an ihrem oberen Ende in dem Lagerpunkt des Schwenkgelenkes oder an den Lagerungspunkten des Schwenkgelenkes gelagert ist. Diese Prothese dient insbesondere als Skiprothese. Die Lagerung des Dämpfers in der Gelenkachse führt zu einer unmittelbaren Krafteinleitung in das Prothesengelenkoberteil.

Aufgabe der vorliegenden Erfindung ist es, eine Prothese bereitzustellen, die insbesondere für große Stumpflängen geeignet ist, so dass auch Exartikulationspatienten eine verbesserte prothetische Versorgung erhalten können.

Erfindungsgemäß wird diese Aufgabe durch eine Prothese mit den Merkmalen des Hauptanspruches gelöst. Vorteilhafte Ausgestaltungen und Weiterbildungen der Erfindung sind in den Unteransprüchen, der Beschreibung sowie den Figuren offenbart. Die Prothese mit einem Trägerteil, das zur Befestigung eines Aufnahmeelementes dienen kann, sieht vor, dass an dem Trägerteil ein Schwenkgelenk befestigt oder ausgebildet ist, über das eine distale Prothesenkomponente an dem Trägerteil befestigt ist. Das Schwenkgelenk ermöglicht eine Flexion und/oder Extension der distalen Prothesenkomponente relativ zu dem Aufnahmeelement oder dem Trägerteil um eine Gelenkachse. Eine Dämpfereinrichtung zur Dämpfung der Flexion und/oder Extension der distalen Prothesenkomponente ist zwischen der distalen Prothesenkomponente und dem Trägerteil oder dem Aufnahmeelement angeordnet. Alternativ oder ergänzend zu der Dämpfereinrichtung können eine Federeinrichtung oder einer Aktuatoreneinrichtung, beispielsweise ein Motor, ein Linearantrieb, ein Magnetantrieb oder dergleichen zwischen der distalen Prothesenkomponente und dem Trägerteil angeordnet, um Kräfte zu speichern, umzuwandeln oder mechanische Energie in das Gelenk einzuspeisen. Die Dämpfereinrichtung, Federeinrichtung und/oder Aktuatoreneinrichtung oder eine Kombination davon, ist mit einem ersten Lagerpunkt an der distalen Prothesenkomponente gelagert und mit einem zweiten Lagerpunkt an einem Hebel oder an einem Lenker. Der Hebel ist dabei schwenkbar um eine Schwenkachse an der distalen Prothesenkomponente gelagert. Der Lenker ist mit einem distalen Abschnitt an dem Hebel und einem proximalen Abschnitt an dem Trägerteil oder dem Aufnahmeelement gelagert. Die Gelenkachse, die zwischen dem Trägerteil bzw. dem Aufnahmeelement und der distalen Prothesenkomponente ausgebildet ist, geht durch das Trägerteil oder das Aufnahmeelement hindurch, so dass es möglich ist, dass die Gelenkachse im Bereich einer natürlichen Gelenkachse einer Gliedmaße positioniert ist oder positioniert werden kann. Die Gelenkachse kann dabei proximal zu einem distalen Ende des Trägerteils oder des Aufnahmeelementes positioniert sein und werden, was Vorteile im Hinblick auf die jeweilige Anordnung der Gelenkachse bei langen Stümpfen mit sich bringt.

Durch die Anlenkung der Dämpfereinrichtung, Federeinrichtung und/oder Aktuatoreneinrichtung über ein Hebelgelenk ist es möglich, relativ kleine Dämpfereinrichtungen, Federeinrichtung und/oder Aktuatoreneinrichtung zu verwenden und die Hebelübersetzung auszunutzen, so dass der relativ große Verschwenkweg des Trägerteils oder des Aufnahmeelementes relativ zu der distalen Prothesenkomponente verringert wird. Wird ein großer Verlagerungsweg benötigt, beispielsweise für Federspeicher, ist dies über eine entsprechende Hebelübersetzung ebenfalls einstellbar. Darüber hinaus ist es möglich, durch die Anlenkung der Dämpfereinrichtung, Federeinrichtung und/oder Aktuatoreneinrichtung in der distalen Prothesenkomponente einen Lagerungspunkt frei zu wählen, so dass Standardkomponenten wie Dämpfer, Federn oder Motoren etc. eingesetzt werden können, die allein über die Anpassung der Hebellänge, Hebellängen oder Lenkerlängen sowie die Anordnung der jeweiligen Schwenkachsen leicht an die jeweils individuell anpassbare Prothese angepasst werden können. Über die Hebelanordnung kann die Dämpferrichtung, Federeinrichtung und/oder Aktuatoreneinrichtung innerhalb des distalen Prothesenelementes geometrisch günstig, insbesondere bauraumoptimiert positioniert werden.

Da die Prothese gemäß dem Stand der Technik eine Lagerung der Dämpfereinrichtung in der Gelenkachse des Gelenkes vorsah, ist es notwendig, dass die Gelenkachse distal über das Stumpfende oder das distale Ende des Aufnahmeelementes hinaussteht oder an dem distalen Ende des Trägerteils angeordnet ist, das wiederum über das Stumpfende hinaussteht. Dies hat zur Folge, dass die Gelenkachse gemäß dem Stand der Technik weit distal zur natürlichen Gelenkachse angeordnet werden muss, wenn ein relativ langer Stumpf erhalten werden konnte, beispielsweise bei Gelenkexartikulationen, insbesondere bei Knieexartikulationen. Ist der Stumpf mit einer knöchernen Struktur mit Kondyle ausgebildet, kann dies hinsichtlich der Belastbarkeit des Stumpfendes vorteilhaft sein. Bei einer Versorgung mit einer Prothese gemäß dem Stand der Technik würde dies eine Längenveränderung des versorgten Beines im Oberschenkelbereich bedeuten, was insbesondere im sitzenden Zustand deutlich sichtbar wäre. Mit der erfindungsgemäßen Lösung ist es möglich, die Gelenkachse nahezu frei zu wählen, insbesondere so anzuordnen, dass die Stumpflänge minimiert wird. Dadurch ergibt sich die Möglichkeit die versorgte Gliedmaße mit einer der unversorgten Gliedmaße entsprechenden Länge der Komponenten auszugestalten. Bei einer Knieexartikulationsprothese hat dies den Vorteil, dass die Oberschenkellänge nicht über die eines unversorgten Beines hinausgeht, was bei öffentlichen Verkehrsmitteln ein Problem darstellen kann. Gleiches gilt für Flugzeuge, Autos sowie Restaurantbesuche oder dergleichen, bei denen durch im Vergleich zur natürlichen Gliedmaße verlängerten Gliedmaßenabschnitten, beispielsweise Oberschenkeln, Behinderungen auftreten oder ungewollte Kollisionen stattfinden.

Über die mit der erfindungsgemäße Prothese erreichbaren gleichlangen Gliedma-ßenkomponenten wie Oberschenkel und Unterschenkel hinsichtlich versorgter und unversorgter Seiten ist es möglich, mit herkömmlichen Fahrrädern zu fahren, ohne dass eine Anpassung der Kurbellängen erfolgen muss. Das Hinknien kann gleichmäßig erfolgen, was bei ungleichmäßigen Oberschenkellängen nur schief möglich und in der Regel mit Hüftschmerzen einhergeht. Bei Prothesen der oberen Extremitäten ist insbesondere die Auffälligkeit der unterschiedlichen Oberarmlängen vermindert, bei dem Einsatz bei Prothesenfüßen ermöglicht die Prothese einen gedämpften Prothesenfuß auch bei langen Unterschenkelstumpflängen, die anders nicht mit einem gedämpften Prothesenfuß versorgt werden könnten.

Das Aufnahmeelement kann zur Aufnahme eines Stumpfes ausgebildet und an dem Träger befestigt sein, wobei das Aufnahmeelement als herkömmlicher Stumpfschaft, als Liner-Schaft-System und/oder als distale Tasse ausgebildet sein, die über ein Gestänge oder eine Stumpfaufnahme an dem Stumpf festlegbar ist. An dem Trägerteil kann das Aufnahmeelement über ein Verriegelungssystem befestigt sein, beispielsweise über ein Pin-System oder ein Shuttle-lock. Das Aufnahmeelement kann als Liner ausgebildet und direkt an dem Trägerteil befestigt sein. Das Trägerteil selbst kann als Stumpfaufnahme ausgeführt sein und den Stumpf unmittelbar oder mit einem Liner aufnehmen. Der Liner kann über Unterdruck und/oder Formschlusselemente an dem Trägerteil befestigt werden. Grundsätzlich ist es auch möglich, dass an dem Trägerteil eine osseointegrierbare Komponente ausgebildet oder befestigt und in dem Knochen verankerbar ist.

Der Hebel kann als zweiarmiger Hebel ausgebildet sein, wobei die Schwenkachse zwischen der Dämpfereinrichtung und dem Lenker positioniert ist. Dadurch ist es möglich, dass Hebelverhältnis und damit das Wegverhältnis zwischen der Dämpfereinrichtung und dem Lenker einfach und vielfältig einzustellen, was sich in der Wegübersetzung und damit auch in der maximalen Weglänge der Dämpfereinrichtung, Federeinrichtung und/oder Aktuatoreneinrichtung niederschlägt. Je größer das Hebelverhältnis ist, desto geringer ist der Verlagerungsweg der Dämpfereinrichtung, Federeinrichtung und/oder Aktuatoreneinrichtung. Die Dämpfereinrichtung ist vorteilhafterweise als Hydraulikdämpfer oder Pneumatikdämpfer oder einer Kombination aus Hydraulikdämpfer und Pneumatikdämpfer ausgebildet.

Alternativ zu einer zweiarmigen Ausgestaltung als Hebel, ähnlich einer Wippe, kann diese auch als einarmiger Hebel ausgebildet sein, wobei der Lenker und die Dämpfereinrichtung, Federeinrichtung und/oder Aktuatoreneinrichtung entfernt von der Schwenkachse an dem Hebel gelagert sind, sowohl der Lenker als auch die Dämpfereinrichtung, Federeinrichtung und/oder Aktuatoreneinrichtung sind an dem einen Hebel gelagert, was eine stabile Zuordnung ermöglicht und einen relativ geringen Bauraum erfordert.

Der Lenker und/oder die Dämpfereinrichtung, Federeinrichtung und/oder Aktuatoreneinrichtung können verlagerbar an dem Hebel gelagert sein, so dass eine Anpassung der Hebelverhältnisse zwischen dem Lenker und der Dämpfereinrichtung, Federeinrichtung und/oder Aktuatoreneinrichtung erfolgen kann, um Notwendigkeiten oder Wünschen der Patienten entgegenzukommen. Dazu können an dem Hebel in diskreten Abständen oder aber auch über Langlöcher Aufnahmen zur Lagerung der Dämpfereinrichtung, Federeinrichtung und/oder Aktuatoreneinrichtung oder des Lenkers angeordnet und ausgebildet sein, so dass eine Anpassung der Position des Lenkers und/oder der Dämpfereinrichtung, Federeinrichtung und/oder Aktuatoreneinrichtung an dem Hebel erfolgen und nach erfolgter Anpassung diese geometrische Zuordnung fixiert werden kann. Die Beweglichkeit des Lenkers und der Dämpfereinrichtung, Federeinrichtung und/oder Aktuatoreneinrichtung an dem Hebel ist aufgrund der Lagerungssituation weiterhin gegeben.

Sowohl der Lenker als auch die Dämpfereinrichtung, Federeinrichtung und/oder Aktuatoreneinrichtung oder der Hebel können längenveränderlich ausgebildet sein, beispielsweise über Schraubhülsen oder teleskopierbare Aufnahmen, die in der jeweils gewünschten Position wieder fixierbar sind, so dass eine Vielzahl von Einstellungen hinsichtlich der Länge und damit auch der Hebelverhältnisse zwischen der Dämpfereinrichtung, Federeinrichtung und/oder Aktuatoreneinrichtung, dem Lenker und dem Hebel möglich und vorgesehen sind.

Die distale Prothesenkomponente kann einen Hohlraum ausbilden, in dem der Hebel und die Dämpfereinrichtung, Federeinrichtung und/oder Aktuatoreneinrichtung angeordnet sind. Über diesen Hohlraum, beispielsweise in Gestalt der fehlenden distalen Gliedmaße, beispielsweise einem Unterarm oder einem Unterschenkel, können die mechanischen Komponenten der Prothese untergebracht werden. Die distale Prothesenkomponente kann die Form der jeweiligen Gliedmaße aufweisen, die es ersetzen soll. Durch die Ausgestaltung des Hohlraumes in der distalen Prothesenkomponente wird einerseits eine angenähert natürliche Anmutung und andererseits ein geschützter Raum für die beweglichen mechanischen Komponenten geschaffen. Die distale Prothesenkomponente bietet somit auch einen mechanischen Schutz der Hebelanordnung sowie der Dämpfereinrichtung, Federeinrichtung und/oder Aktuatoreneinrichtung und weiteren Komponenten wie Sensoren oder einer Steuerung.

Eine vorteilhafte Weiterbildung der Erfindung sieht vor, dass der Lenker den Zugang zu dem Hohlraum zumindest teilweise abdeckt oder versperrt. Der Hohlraum kann insbesondere in dem Bereich, in dem die distale Prothesenkomponente sich dem Trägerteil oder dem Aufnahmeelement annähert, eine Ausnehmung aufweisen, durch die der Lenker in den Hohlraum der distalen Prothesenkomponente eindringen und mit dem Hebel wechselwirken kann. Der Hebel macht aufgrund der Rotationsbewegung um die Gelenkachse sowie dem Versatz des oberen Lagerungspunktes des Lenkers zu der Gelenkachse bei der Schwenkbewegung der distalen Prothesenkomponente eine Verlagerung auf einer Kreisbahn, was neben einer Längsverlagerung des Lenkers auch eine Rotation um den Lagerungspunkt an dem Hebel zur Folge hat. Dies bedeutet, dass der Lenker relativ zu der distalen Prothesenkomponente auch eine Verschwenkbewegung durchführt, was ein relativ großes Durchgangsloch in der distalen Prothesenkomponente erfordert. Durch eine beispielsweise flächige Ausgestaltung des Lenkers oder die Anordnung eines flächigen Verkleidungsteils an dem beispielsweise posterioren Teil des Lenkers ist es möglich, die distale Prothesenkomponente hinsichtlich des Umfanges durch den Lenker zu komplettieren und den Zugang zu dem Hohlraum zumindest teilweise abzudecken, so dass der Lenker neben einer Kraftübertragungsfunktion auch eine Schutzfunktion ausübt. Bei der Ausgestaltung der distalen Prothesenkomponente als Unterschenkelrohr kann der Lenker wadenförmig ausgebildet sein oder die distale Prothesenkomponente in ihrer Form zu einer Wade ergänzen.

Die distale Prothesenkomponente kann unmittelbar an dem Trägerteil oder dem Aufnahmeteil an zumindest einem Lagerpunkt gelagert sein, vorteilhafterweise findet die Lagerung an zwei Lagerpunkten statt, die auf der Gelenkachse liegen. Der zumindest eine Lagerpunkt liegt medial oder lateral an dem Trägerteil oder dem Aufnahmeelement, wenn zwei Lagerpunkte eingesetzt werden, liegen diese medial und lateral an dem Trägerteil und dem Aufnahmeelement.

Das Trägerteil kann als eine Kappe ausgebildet sein, das mit dem Aufnahmeelement in Gestalt eines Schaftes oder mehrerer Schienen verbunden werden kann. In dem Trägerteil kann der Stumpf direkt anliegen oder aufliegen, ebenso ist es möglich, dass das Aufnahmeelement in Gestalt eines festen Außenschaftes das Oberteil des Prothesengelenkes ausbildet und die distale Prothesenkomponente direkt an dem Aufnahmeelement schwenkbar gelagert ist.

Der Lenker kann verstellbar an dem Trägerteil oder dem Aufnahmeelement gelagert sein, wodurch sich verstärkte Einflüsse auf den Verlauf der Dämpferkurve, insbesondere des Umkehrpunktes der Dämpfereinrichtung realisieren lassen.

Nachfolgend werden Ausführungsbeispiele der Erfindung anhand der beigefügten Figuren näher erläutert. Es zeigen:
- Figur 1: eine perspektivische Ansicht einer Prothese;
- Figur 2: eine Seitenansicht einer Prothese ohne dargestellten Dämpfer;
- Figur 3: eine Schnittdarstellung durch eine Prothese in Extensionsstellung;
- Figur 4: eine Prothese gemäß Figur 3 in 90°-Stellung;
- Figur 5: eine Darstellung gemäß Figur 5 in maximalflektierter Stellung;
- Figur 6: Dämpfungsverläufe über den Kniewinkel in Abhängigkeit von der Länge des hydraulischen Dämpfers;
- Figuren 7 bis 14: schematische Darstellungen von Varianten der Erfindung;
- Figur 15: einen zweiteiligen Hebel in einer Grundstellung;
- Figur 16: den Hebel gemäß Figur 15 in einer verstellten Stellung; sowie
- Figur 17: eine Variante eines zweiteiligen Hebels.

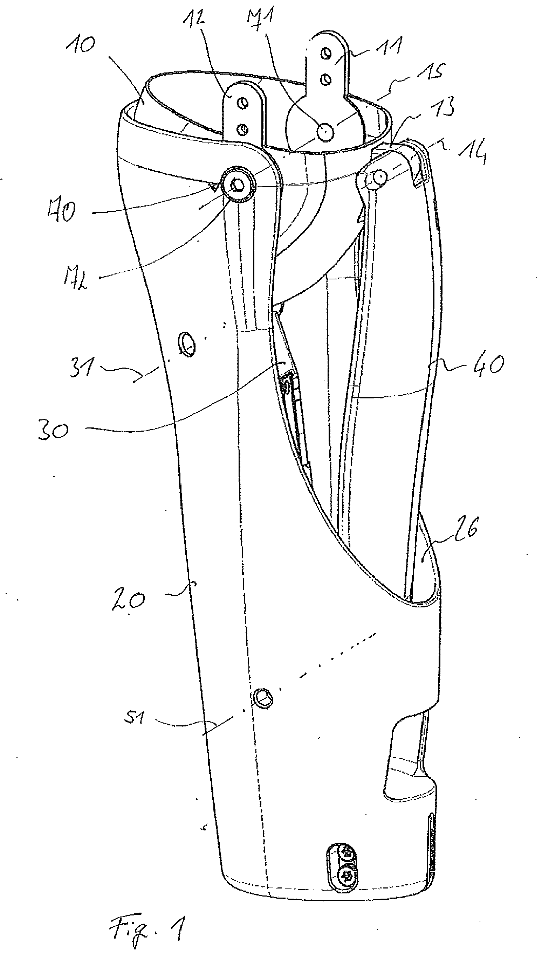

Figur 1 zeigt in einer perspektivischen Darstellung von schräg hinten eine Prothese in Gestalt einer Unterschenkelprothese mit einem Trägerteil 10, an dem ein Aufnahmeelement zur Aufnahme eines Stumpfes ausgebildet oder befestigbar ist. Das Aufnahmeelement kann beispielsweise ein Prothesenschaft mit einem geschlossenen Umfang, Längsstreben, die sich um einen Oberschenkelstumpf schließen und über zirkuläre Zugmittel festgelegt werden oder eine andere Aufnahmeeinrichtung ausgebildet sein. Grundsätzlich ist es auch möglich, dass das Trägerteil 10 dazu ausgebildet ist, den Stumpf oder den Stumpf mit einem Liner aufzunehmen. Zur Befestigung eines separaten Aufnahmeelementes sind mediale und laterale Befestigungslaschen 11, 12 angeordnet, die an dem Trägerteil 10 befestigt sind und über die das Aufnahmeelement oder die Aufnahmeelemente an dem Trägerteil 10 befestigt werden können. Die Befestigung kann über Schrauben, Bolzen oder andere Formschlusselemente erreicht werden, ebenfalls ist es möglich, dass das Aufnahmeelement an oder in dem Trägerteil 10, das eine tassenartige Form aufweist, eingeklebt oder angeklebt oder auf andere Art und Weise formschlüssig oder stoffschlüssig befestigt ist.

Das Trägerteil 10 ist an einer distalen Prothesenkomponente 20, im dargestellten Ausführungsbeispiel ein Unterschenkelteil, schwenkbar um eine Gelenkachse 15 befestigt. Die Gelenkachse 15 erstreckt sich von medial nach lateral und vorzugsweise durch die Laschen 11, 12. Zur schwenkbaren Befestigung des Trägerteils 10 an der distalen Prothesenkomponente 20 sind medial und lateral an dem als Hohlkörper ausgebildeten distalen Prothesenelement 20 Lagerungsstellen 72, 71 ausgebildet, so dass zwischen dem Trägerteil 10 und der distalen Prothesenkomponente 20 ein Schwenkgelenk 70 ausgebildet wird.

Innerhalb der distalen Prothesenkomponente 20 ist eine Dämpfereinrichtung 30 angeordnet, ergänzend oder alternativ zu der Dämpfereinrichtung 30 können eine Federeinrichtung, eine Aktuatoreneinrichtung oder eine Kombination wenigstens zweier dieser Einrichtungen angeordnet sein. Die dargestellte Dämpfereinrichtung 30 ist als Hydraulikdämpfer ausgebildet und weist einen proximalen, das heißt in der Nähe des Schwenkgelenkes 30 befestigten oberen Lagerungspunkt auf, der später näher erläutert wird. Der obere oder proximale Lagerungspunkt bildet eine Lagerachse 31 aus, die im Wesentlichen parallel zu der Gelenkachse 15 orientiert ist. Wird nachfolgend von der Dämpfereinrichtung gesprochen, gelten die Ausführungen auch für Federeinrichtungen und/oder Aktuatoreneinrichtungen oder Kombinationen auch zwei oder allen Einrichtungen.

An dem Trägerteil 10 ist an einer hinteren posterioren Seite eine Lenkeraufnahme 13 ausgebildet, die zur schwenkbaren Aufnahme um eine Achse 14 für einen posterior angeordneten Lenker 40 dient. Die Achse 14 ist beabstandet zu der Gelenkachse 15 angeordnet, so dass bei einer Rotation des Trägerteils 10 um die Gelenkachse 15 der Lenker 40 mit seinem oberen Ende eine Bahnbewegung entlang der Bahn der Achse 14 ausführt.

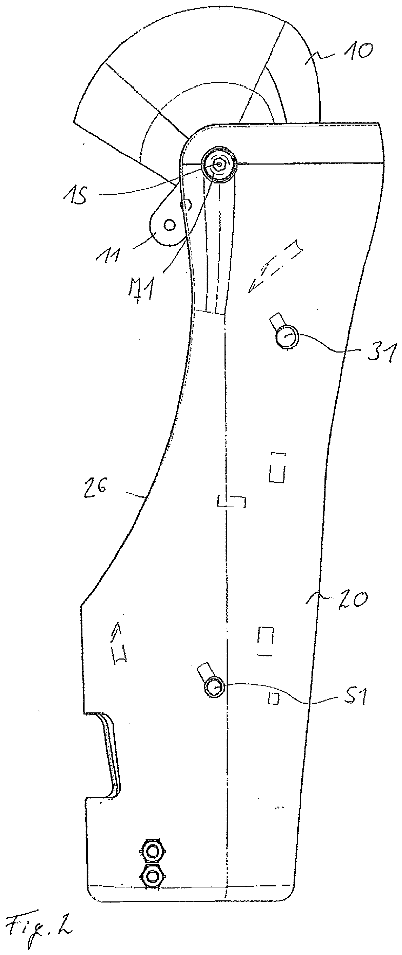

Figur 2 zeigt die Prothese in einer Seitenansicht ohne den hinteren Lenker 40, an der distalen Prothesenkomponente 20 sind eine obere Achse 31 für den oberen Lagerpunkt der Dämpfereinrichtung 30 sowie eine untere Achse 51 für einen nachfolgend erläuterten Hebel zu erkennen. Beide Achsen 31, 51 sind verlagerbar in oder an der distalen Prothesenkomponente 20 gelagert, so dass sowohl der Abstand als auch die Position der jeweiligen Achsen 31, 51 einstellbar und in der eingestellten Position festlegbar ist.

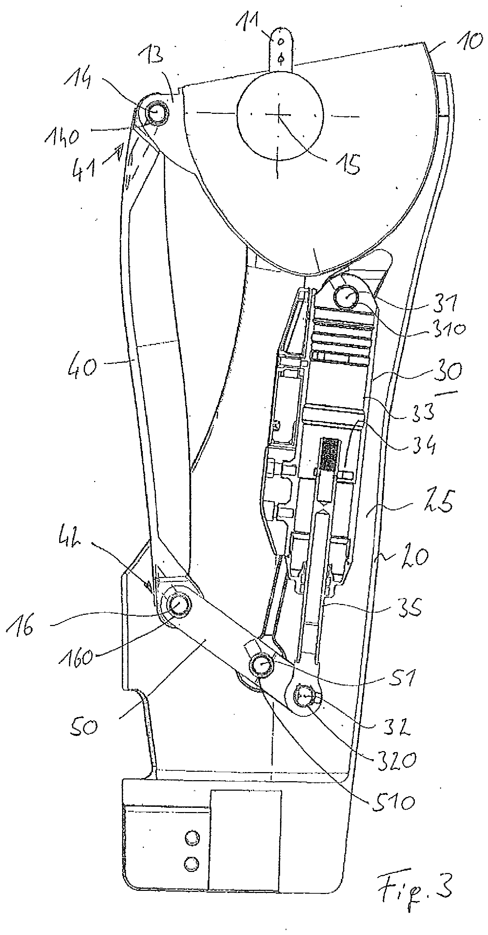

In der Figur 3 ist in einer Schnittdarstellung die Prothese gemäß Figur 1 gezeigt. Das schalenartige Trägerteil 10 kann zur Aufnahme eines Oberarmstumpfes, Unterschenkelstumpfes oder Oberschenkelstumpfes ausgebildet sein. An dem hinteren, posterioren Wandabschnitt des Trägerteils 10 ist die Lenkeraufnahme 13 zu erkennen, der zur Aufnahme eines Bolzens zur Ausbildung einer Lagerstelle 41 zur gelenkigen Befestigung des hinteren Lenkers 40 an dem Trägerteil 10 zu erkennen ist. Der Lenker 40 ist in seinem proximalen Bereich 41, präziser an seinem proximalen Ende, an der Lagerstelle 140 des Trägerteils 10 festgelegt. An dem gegenüberliegenden Ende ist der Lenker 40 in einem distalen Bereich 42, im dargestellten Ausführungsbeispiel mit seinem distalen Ende, an einem Hebel 50 gelagert. Der Hebel 50 ist um eine Schwenkachse 51 an dem distalen Prothesenelement 20 gelagert und als zweiarmiger Hebel ausgebildet. Der hintere Lenker 40 bildet mit dem Hebel 50 eine Lagerstelle 160 aus, so dass eine Verschwenkung des Hebels 50 bei einer Verlagerung des Lenkers 40 um die Achse 16 möglich ist.

Auf dem dem Lenker 40 abgewandten Hebelende, das auf der anderen Seite der Achse 51 liegt, ist die Dämpfereinheit 30 an dem unteren Lagerungspunkt 320 festgelegt. Der Hebel 50 selbst ist an der Lagerstelle 510 innerhalb des durch das distale Prothesenelement 20 gebildeten Hohlraums 25 schwenkbar gelagert. Die Lagerstelle 320 an dem Hebel 50 ermöglicht eine Schwenkbewegung zwischen einer Kolbenstange 35 und dem Hebel 50, so dass bei einer Drehung um die Achse 51 die Kolbenstange eine Linearbewegung ausführen kann, so dass der innerhalb eines Zylinders 33 angeordnete Kolben 34 der als Hydraulikdämpfer ausgebildeten Dämpfereinheit 40 eine Verlagerungsbewegung ausführen kann. Das obere, proximale Ende der Dämpfereinrichtung 30 ist an der oberen Lagerstelle 310 verschwenkbar um die Achse 31 gelagert.

Neben der festen Zuordnung der Komponenten zueinander, wie sie in der Figur 3 dargestellt ist, besteht die Möglichkeit, dass die Lagerungsstellen 140, 160, 320 verstellbar ausgestaltetet sind, so dass die jeweiligen Komponenten an unterschiedlichen Positionen schwenkbar festlegbar sind. Insbesondere können die Abstände der Lagerungsstellen 160, 320 des Lenkers 40 oder der Dämpfereinheit 30 relativ zu der zwischen den beiden Lagerungspunkten 160, 320 angeordneten Schwenkachse 51 verändert werden, so dass sich das Übersetzungsverhältnis verändert. Auch ist es möglich, unterschiedlich lange Lenker 40 zwischen den Lagerstellen 140, 160 anzuordnen oder den Lenker 40 längenverstellbar auszugestalten, beispielsweise über Schraubhülsen, zueinander verlagerbare Komponenten, eine teleskopierbare Ausgestaltung oder über Zwischenstücke. Dadurch ist es möglich, die Charakteristik der Dämpfung, eine Verlagerung eines Umkehrpunktes der Dämpfereinrichtung 30 sowie andere Kraftübersetzungen zu realisieren.

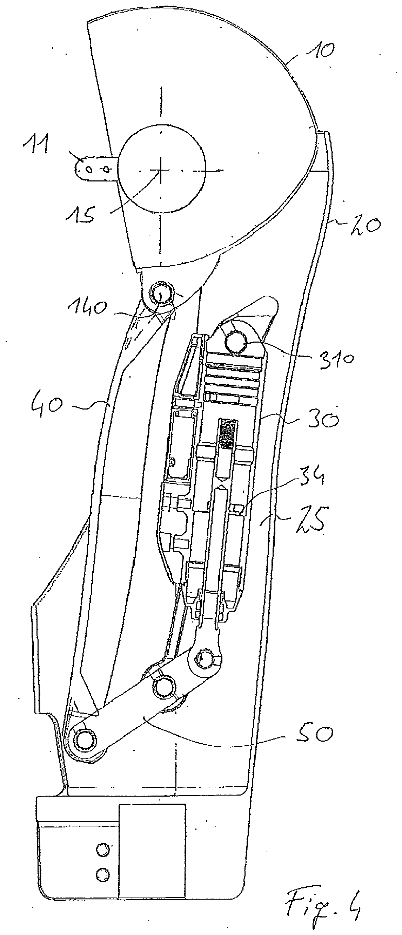

Figur 4 zeigt die Ausgestaltung der Prothese in einer abgewinkelten Stellung, bei der das Trägerteil 10 um ca. 90° zu einer maximal extendierten Stellung relativ zum distalen Prothesenelement 20 flektiert ist. Aufgrund der Bahnkurve der oberen Lagerungsstelle 140 des Lenkers 40 wird der Lenker 40 einerseits um die Achse 14 verschwenkt und andererseits das distale Ende nach unten bewegt, was dazu führt, dass der Hebel 50 um die Schwenkachse 51 verschwenkt und das gegenüberliegende Hebelende nach oben in Richtung auf die obere Lagerungsstelle 310 verlagert wird. Dadurch wird die Kolbenstange 35 in den Hydraulikdämpfer eingefahren und der Kolben 34 innerhalb des Zylinders 33 verlagert.

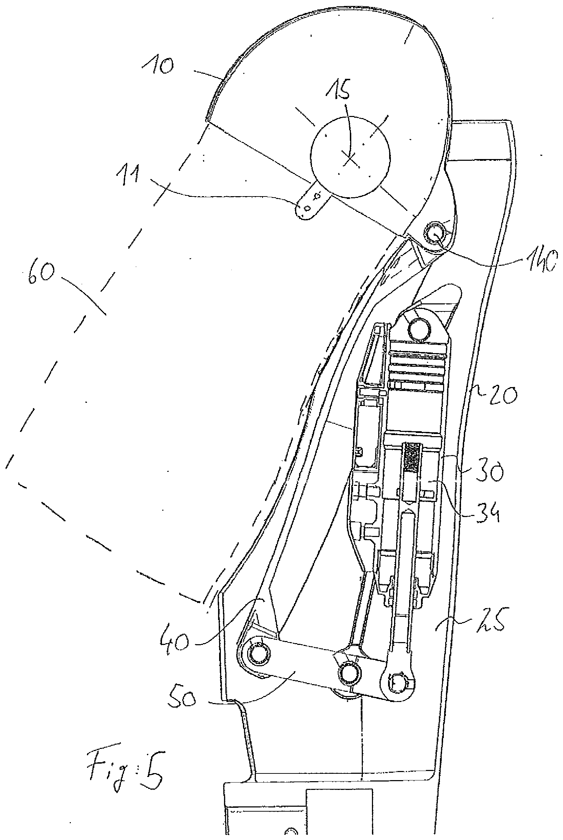

In Figur 5 ist die Prothese in einer maximal flektierten Stellung gezeigt. Das Trägerteil 10 ist um die Gelenkachse 15 maximal verschwenkt, so dass das angedeutete Aufnahmeelement 60 in Gestalt eines Oberschenkelschaftes an der Rückseite des distalen Prothesenelementes 20 anliegt. Der Lenker 40 ist aufgrund der Bewegungsumkehr der oberen Lagerstelle 140 wieder in Proximalrichtung verlagert, so dass der Hebel 50 in die entgegengesetzte Richtung, also im Uhrzeigersinn verschwenkt wurde, ausgehend von der Stellung gemäß Figur 4. Dadurch ergibt sich eine Bewegungsumkehr des Kolbens 34 in der Dämpfereinrichtung 30. Durch eine unterschiedliche Widerstandswahl bei Überströmventilen in der jeweiligen Bewegungsrichtung ist es möglich, eine angepasste Widerstandsaufbringung bereitzustellen. Bei einer Bewegung bis zur Stellung gemäß Figur 4, also bei einer Flexion aus einer extendierten Stellung in eine ungefähr 90°-Stellung verfährt der Kolben 34 nach oben. In der Stellung gemäß Figur 4 hat er seine Maximalstellung erreicht, bei der Bewegungsumkehr bei einer weiteren Flexion oder auch einer Extension wird der Kolben 34 nach unten bewegt und kann beispielsweise einen geringeren Widerstand gegen eine weitere Einbeugung bereitstellen als bei einer Bewegung nach oben.

Statt einer Dämpfereinrichtung 30 ist es möglich, eine Federeinrichtung, eine Aktuatoreneinrichtung beispielsweise in Gestalt eines Motors oder eine Kombination wenigstens zweier dieser Einrichtungen innerhalb des Hohlraumes 25 anzuordnen. Der Hebel 50 kann längenveränderlich sein, ebenso kann die Kolbenstange 35 längenveränderlich sein, beispielsweise durch Schraubhülsen. Auch der Lenker 40 kann längenveränderbar ausgebildet sein.

Neben einer Verstellung der Lagerstellen 140, 160, 320 ist es möglich, auch die Lagerstellen 520, 310 des Hebels 50 und der Dämpfereinrichtung 30 verstellbar auszugestalten, also die Lagerstellen 510, 310 an der distalen Prothesenkomponente 20 an unterschiedlichen Stellen festzulegen und dort zu fixieren, um den dynamischen und statischen Aufbau der Prothese zu beeinflussen.

In der Ausführungsform gemäß der Figuren 1 bis 5 ist die Prothese als AKP (above knee prosthesis) ausgebildet, insbesondere ist die Ausgestaltung für Patienten mit einer Knieexartikulation geeignet, da der Stumpf in das Trägerteil 40 hineinragen kann, so dass die Gelenkachse 15 des Schwenkgelenks 70, das zwischen dem Trägerteil 10 und der distalen Prothesenkomponente 20 ausgebildet ist, im Bereich der natürlichen Gelenkachse des gesunden Beines anordenbar ist. Die Gelenkachse 15 kann durch die Positionierung der Lagerstellen 71, 72 an dem Trägerteil 10 oder direkt an dem Aufnahmeelement 50 individuell angepasst werden. Das schalenförmige Unterteil in Gestalt der distalen Prothesenkomponente 20 umgreift teilweise das Trägerteil 10 und bildet korrespondierende Lagerstellen an den medialen und lateralen Seitenwänden aus. Da Knieexartikulationsprothesen im proximalen Bereich nicht über Pyramidenaufnahmeelemente verstellt werden können, ist es notwendig, dass zur Einstellung des dynamischen und statischen Aufbaus der Prothese die Lagerpunkte verstellbar sind, um eine Vor- und Rückverlagerung und dadurch den Prothesenaufbau einzustellen. Es ist beispielsweise möglich, den Hebel 50 selbst mehrteilig auszubilden und verschwenkbar auszugestalten, so dass sich statt der geraden, zweiarmigen Ausgestaltung, wie er in der Figur 3 zu erkennen ist, eine abgewinkelte Anordnung der beiden jenseits der Schwenkachse 51 positionierten Hebelschenkel einstellt. Durch eine Winkelstellung der jeweiligen Hebelschenkel ist es möglich, einerseits den Abstand zwischen den Lagerungspunkten 160, 320 des Lenkers 40 und der Kolbenstange 35 zu verändern und darüber hinaus eine wirksame Veränderung der Umkehrpunkte und damit eine wirksame Variation der Dämpfereigenschaften zu ermöglichen.

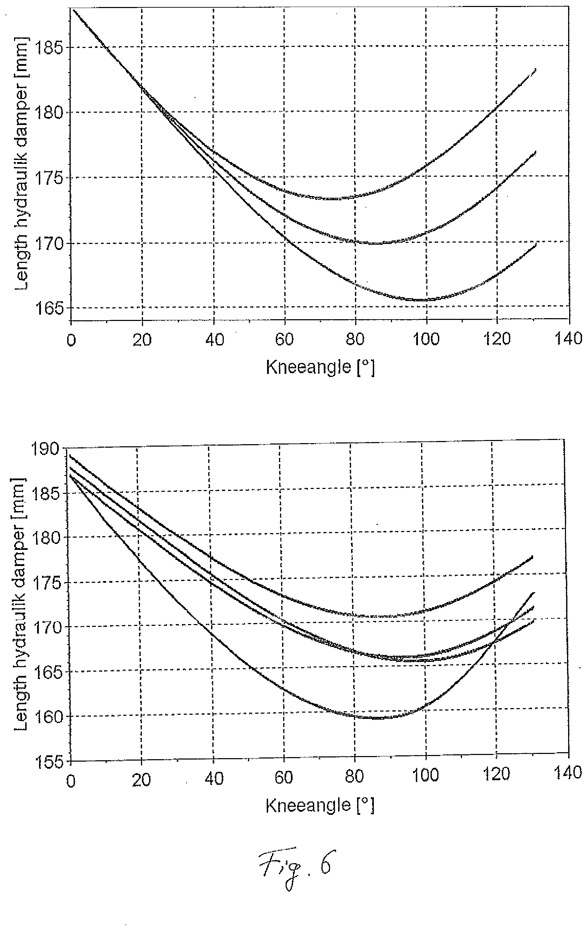

Figur 6 zeigt die Abhängigkeit des Dämpferkraftverlaufes über den Gelenkwinkel, insbesondere Kniewinkel, bei unterschiedlichen Längen des Hydraulikdämpfers. In der oberen Darstellung zeigt die obere Kurve einen Dämpfungsverlauf bei einem langen Lenker 40, der mittlere Verlauf zeigt einen kürzeren und der untere Verlauf den kürzesten Lenker 40. Je nach Lenkerlänge wird der Umkehrpunkt des Kolbens bei unterschiedlichen Winkelstellungen erreicht. Je länger der Lenker 40 ist, desto früher wird der Umkehrpunkt, also der tiefste Punkt der Kurve, erreicht. Die Dämpfung nimmt im Bereich des Umkehrpunktes ab, so dass es vorteilhaft ist, wenn der Totpunkt, also der Umkehrpunkt des Kolbens, im Bereich einer Einbeugung von 90° erreicht wird.

Die untere Darstellung zeigt den Dämpferverlauf bei unterschiedlich angeordneten Achsen und Abständen sowie Hebelverhältnissen. Es ist zu erkennen, dass bei unterschiedlichen Kniewinkeln unterschiedliche Dämpfereigenschaften vorhanden sind.

Über die Veränderung der Hebellängen, insbesondere der Lenkerlänge und der Stellung der Kolbenstange 35 ist es möglich, den Streckanschlag der Prothese zu verstellen. Dies kann an der Dämpfereinheit 30, beispielsweise an dem Hydraulikdämpfer durch die Verstellung der Länge der Kolbenstange 35 oder die Verstellung der Länge des Lenkers 40 erreicht werden. Auch ist es möglich, durch eine Zuordnung der verdrehbar zueinander gelagerten Hebelschenkel des Hebels 50 eine entsprechende Variierung des Streckanschlages sowie des maximalen Flexionswinkels und/oder Extensionswinkels zu erreichen.

Die Dämpfungseigenschaften der Dämpfereinrichtung 30 werden bevorzugt über den Kniewinkel so eingestellt, dass sie für die Funktionalität der Prothese optimiert sind. Im Bereich des Totpunktes oder des Umlenkpunktes des Kolbens kann die Dämpfung erhöht werden, damit die Dämpfungsmomente oder Drehmomente in dem Schwenkgelenk 70 nicht schlagartig wegfallen.

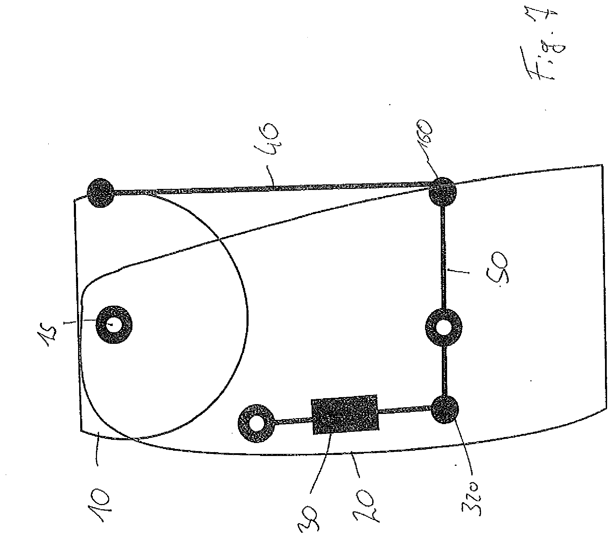

Die Figur 7 zeigt eine schematische Darstellung der Komponenten gemäß der Anordnung der Figuren 3 bis 5. Es ist zu erkennen, dass die Gelenkachse 15 proximal zu dem distalen Ende des Trägerteils 10 angeordnet ist, so dass eine einfache Längenkorrektur bei langen Stümpfen stattfinden kann, da die Lagerungspunkte nahezu beliebig an dem Trägerteil 10 angeordnet und die Lage der Gelenkachse 15 an die der natürlichen Gelenkachse angepasst werden können. Neben der dargestellten Ausführungsform mit einer Knieexartikulationsprothese ist es auch möglich, solche Prothesen an Unterschenkeln oder Oberarmen zu positionieren. Der Hebel 50 ist als zweiarmiger Hebel ausgebildet, der hintere Lenker 40 ist an einem Hebelende angeordnet, die Dämpfereinrichtung 30, ebenfalls zusammen mit einer Federeinrichtung oder einer Aktuatoreinheit, ist an dem gegenüberliegenden Hebelende des zweiarmigen Hebels 50, der um die Schwenkachse 51 verlagerbar ist, angeordnet. Die Lagerungspunkte sowohl des Hebels 50 als auch der Dämpfereinheit 30 und/oder des Lenkers 40 können verstellbar sein.

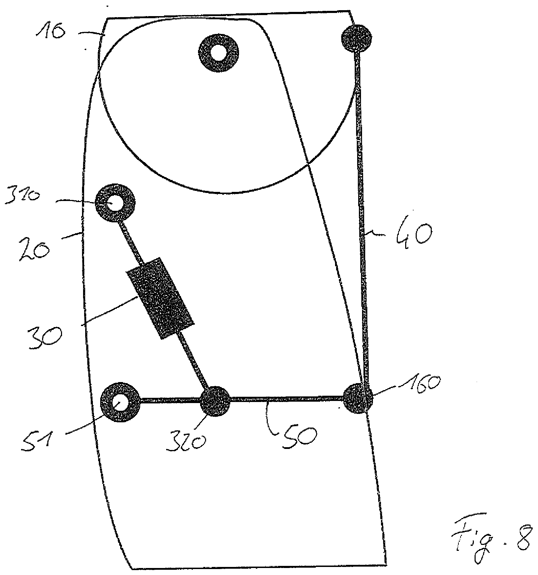

Figur 8 zeigt eine Variante der Anordnung mit einem einarmigen Hebel 50, bei dem die Lagerungspunkte 160, 320 des Lenkers 40 auf der gleichen Seite der Schwenkachse 51 liegen. Über eine Beabstandung der Lagerungspunkte 160, 320 zueinander und der Einstellung des Abstandes zueinander ist es möglich, das Weg- und Kraft-übersetzungsverhältnis zu verändern. Der obere Lagerungspunkt 310 der Dämpfereinrichtung 30 liegt im dargestellten Ausführungsbeispiel oberhalb der Schwenkachse 51 und des Hebels 50.

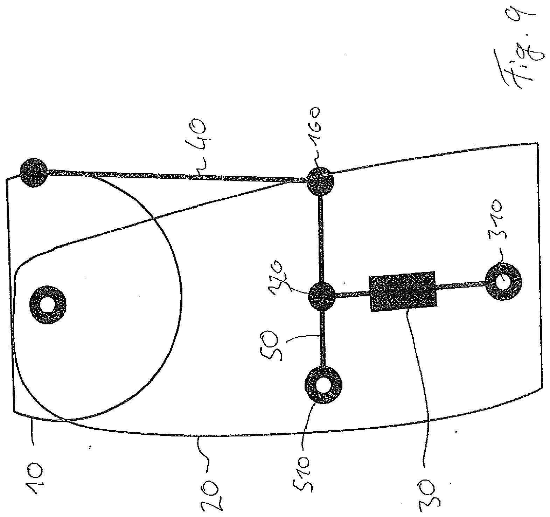

Eine Variante der Erfindung ist in der Figur 9 dargestellt, die einen ähnlichen Aufbau wie die Figur 8, allerdings ist der zweite Lagerungspunkt 310 der Dämpfereinheit 30 nicht oberhalb des Hebels 50, sondern unterhalb des Hebels 50 an der distalen Prothesenkomponente 20 angeordnet. Eine solche Anordnung ist sinnvoll, wenn die Schwenkachse 51 relativ weit proximal im distalen Prothesenelement 30 angeordnet ist und zum Trägerteil 10 nur noch ein geringer Abstand vorliegt. Auch hier können die Lagerungspunkte 510, 320, 310 und 160 verschieblich oder veränderlich ausgestaltet sein.

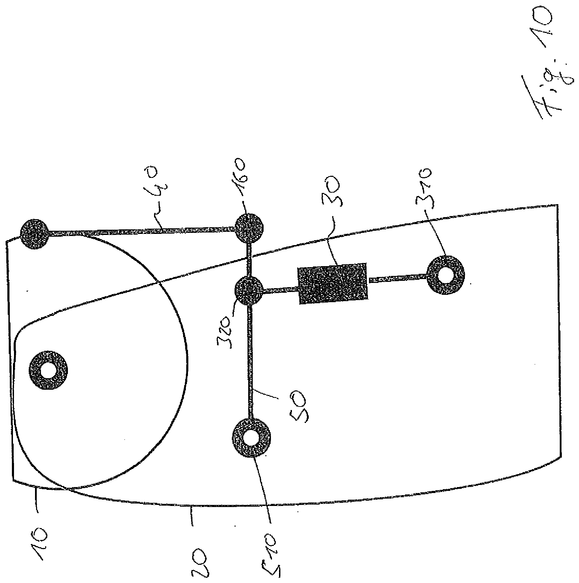

Eine Variante der Ausgestaltung gemäß Figur 9 ist in der Figur 10 dargestellt, bei der der distale Lagerungspunkt 310 weiter posterior angeordnet ist, der Lagerungspunkt 510 des Hebels ist sehr weit oben angeordnet und aufgrund der vergleichsweise geringen Abstände zwischen dem Lagerungspunkt 320 auf dem Hebel und dem Lagerungspunkt 160 des Lenkers 40 ergibt sich eine geringe Kraft- und Wegübersetzung.

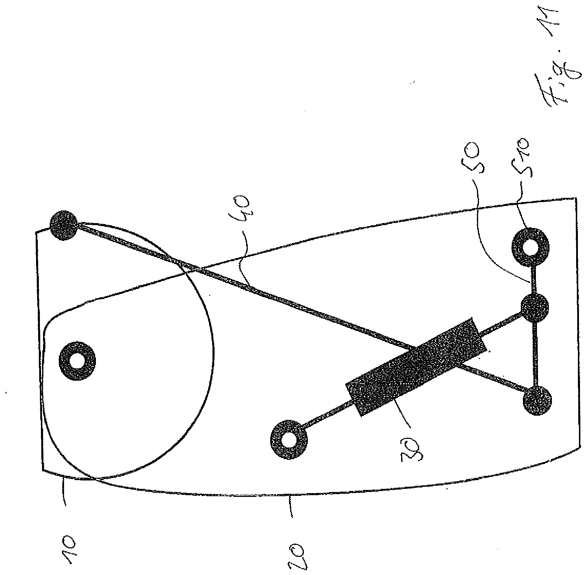

Figur 11 zeigt eine weitere Variante mit einer sich überkreuzenden Führung der Längsachsen der Lenker 40 und der Dämpfereinrichtung 30. Auch hier ist der Hebel 50 als einarmiger Hebel ausgebildet, die Lagerungsstelle 510 liegt im posterioren Bereich am distalen Ende der distalen Prothesenkomponente 20. Wird das Schwenkgelenk flektiert, wandert der Hebel 50 entgegen dem Uhrzeigersinn nach unten, wodurch die Dämpfereinrichtung 30 zugbelastet wird, bei einer Extensionsbewegung wird die Dämpfereinrichtung 30 mit einer Druckkraft beaufschlagt.

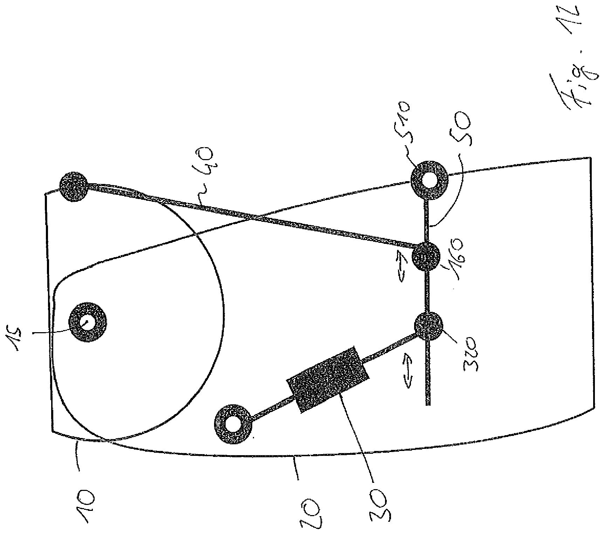

Eine weitere Variante mit einem einarmigen Hebel ist in der Figur 12 dargestellt, der Lagerungspunkt 510 des Hebels 50 liegt posterior der Gelenkachse 15, das freie Hebelende ragt in anteriore Richtung und verschwenkt entgegen dem Uhrzeigersinn, wenn eine Flexion ausgeführt wird. Die Variante gemäß Figur 12 ermöglicht eine Wegvergrößerung für die Dämpfereinrichtung 30. Die Doppelpfeile zeigen die mögliche Verstellbarkeit entlang der Längserstreckung des Hebels 50.

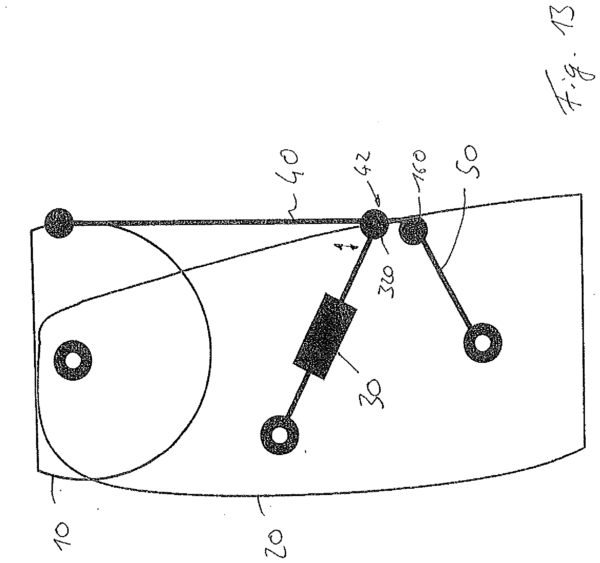

Eine weitere Variante ist in der Figur 13 dargestellt, bei der die Dämpfereinrichtung 30 nicht an dem Hebel 50, sondern an dem distalen Bereich 42 des Lenkers angeordnet ist. Der Hebel 50 bewirkt eine Führung des Lenkers 40 und der Dämpfereinrichtung 30, der Lagerungspunkt 320 liegt an dem Lenker 40 und kann dort verlagerbar festgelegt sein. Über die Winkelorientierung der Längserstreckung der Dämpfereinrichtung 30 kann eine Kraft- oder Wegübersetzung erfolgen.

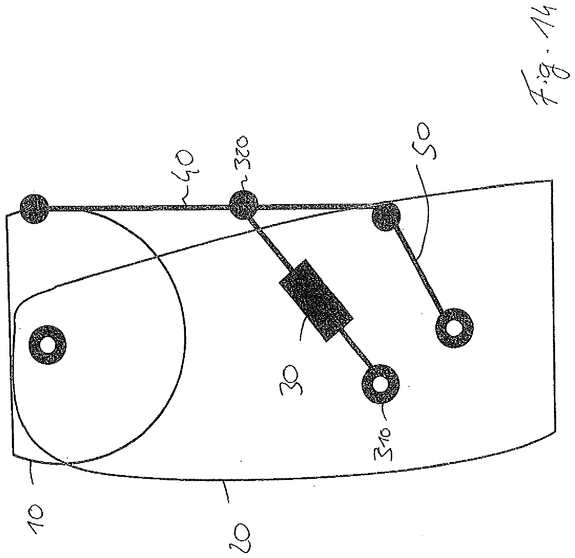

Figur 14 zeigt eine Variante der Figur 13, bei der ebenfalls die Dämpfereinrichtung 30 an dem Lenker 40 befestigt ist, allerdings in einer Variante, bei der die Lagerungsstelle 310 an der distalen Prothesenkomponente 20 unterhalb, also distal zu der Lagerungsstelle 320 der Kolbenstange 35 an dem hinteren Lenker 40 positioniert ist. Bei einer Flexion wird die Dämpfereinrichtung 30 auf Druck belastet, bei einer Extension umgekehrt auf Zug.

Insbesondere den Figuren 1 bis 5 ist zu entnehmen, dass der Lenker 40 den Hohlraum 25, der durch das rohrförmige distale Prothesenelement 20 ausgebildet wird, von posteriorer Seite her zumindest teilweise verschlossen wird. In dem distalen Prothesenelement 20 ist ein Ausschnitt 26 vorgesehen, in dem das Material der Wand entfernt ist, so dass sich eine gebogene Kontur in Seitenansicht ergibt, wie in der Figur 2 dargestellt ist. Dadurch ist es möglich, dass das Trägerteil 10 zusammen mit dem daran befestigten oder daran ausgebildeten Aufnahmeelement 60 eine Flexion durchführen kann, die über 90° hinausgeht. Dies ist in der Figur 5 dargestellt. Der Lenker 40 ist posterior vor dem Ausschnitt 26 angeordnet und bildet eine Abdeckung für die Dämpfereinheit 30 sowie die Mechanik und kann als konturergänzendes Bauteil der Prothese dienen.

Die Dämpfereinheit 30 gegebenenfalls in Verbindung mit einer Federeinheit oder einer Aktuatoreneinheit, die als Motor ausgebildet ist und eine unterstützende oder bremsende Wirkung für das Prothesengelenk darstellen kann, kann rein mechanisch gesteuert werden. Alternativ dazu ist es möglich, eine Steuerung über einen Kniewinkelsensor und einen Drehmomentsensor am Knöchel bei einer Ausgestaltung als Knieexartikulationsprothese auszuführen. Ebenso ist es möglich, dass die Steuerung über einen Inertialsensor im distalen Prothesenelement 20 in Kombination mit einer Hydraulik, die unter einer Belastung sperrt, ausgeführt wird. Neben einer Kombination mit einer Hydraulik, die unter Belastung sperrt, kann ein Inertialsensor im distalen Prothesenelement in Kombination mit einer Drehmomentmessung an dem Schwenkgelenk 70, einer Kraftmessung an der Kolbenstange oder den Lagerungspunkten der Dämpfereinheit 30 oder über eine Druckmessung im Hydraulikkreislauf erfolgen. Ebenso ist es möglich, Inertialsensoren sowohl im Trägerteil, an dem Aufnahmeelement als auch im distalen Prothesenelement vorzusehen. Als Inertialsensoren können Gyroskope sowie Beschleunigungssensoren und Kombination aus beiden Sensoren eingesetzt werden. Kraftsensoren können als Dehnungsmessstreifen, Drucksensoren als Piezoelemente sowie Winkelsensoren als Hallsensoren ausgebildet sein.

Durch die Ausgestaltung der Prothese mit dem Lenker in Verbindung mit dem Hebel kann der Hub in der Hydraulikeinheit oder in dem Aktuator deutlich reduziert werden. Eine Verringerung des Hubes an der Hydraulik führt dazu, dass sowohl die Kolbenstange als auch das Gehäuse um den verringerten Hub gekürzt werden können, so dass die Hydraulik insgesamt um das Doppelte des reduzierten Hubs verkürzt werden kann. Dies ist insbesondere bei beengten Platzverhältnissen ein sehr großer Vorteil.

Durch die Änderung der Länge des Lenkers 40 oder durch die Verlängerung bzw. Verkürzung der Hydraulik oder der Winkelstellung der Hebelarme zueinander kann die Stellung des Trägerteils 10 und damit auch eines möglichen Prothesenschaftes und damit des Prothesenaufbaus geändert werden.

Durch die Ausgestaltung des Trägerteils als eine Schale ist es möglich, endbelastbare Stümpfe mit neuen Schaftkonzepten festzulegen und den Stumpf direkt in dem Trägerteil 10 aufzunehmen. Die Festlegung der Schwenkachse 70 an dem Trägerteil proximal zu dem distalen Ende des Trägerteils ermöglicht es, die Gelenkachse 15 in proximaler Richtung an den Stumpf zu verschieben oder zumindest möglichst nahe an den Stumpf heran. Dadurch wird das Trägheitsmoment aufgrund des proximalen Drehpunktes oder der proximalen Gelenksachse 15 verbessert, der Patient erhält ein subjektiv leichteres Empfinden für die Prothese.

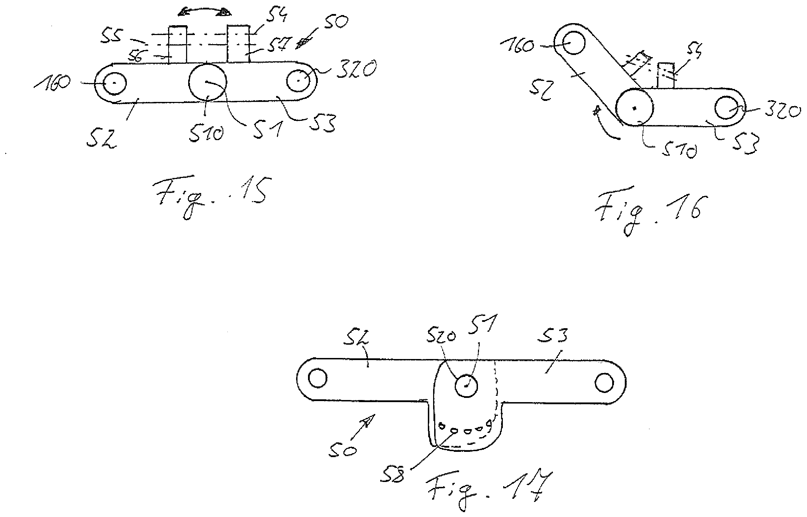

In der Figur 15 ist eine Variante mit einem zweiteiligen Hebel 50 gezeigt, bei dem ein erstes Hebelteil 52 mit einem zweiten Hebelteil 53 um die Schwenkachse 51 verschwenkbar verbunden ist. An jedem Hebelteil 52 oder 51 sind Ausleger 56, 57 angeordnet, die Gewinde bzw. Durchgangslöcher aufweisen und zwischen denen beispielsweise Schrauben 54, 55 angeordnet sind. Die Schrauben 54 oder 55 sind Einstellschrauben, eine der Schrauben zieht die Ausleger 56, 57 aufeinander zu, während die andere Schraube gegenläufig drückt, so dass eine Verspannung der Einstellung erfolgen kann. In der dargestellten Position gemäß Figur 15 sind die jeweiligen Lagerungspunkte 160, 320 maximal voneinander entfernt, die Hebelteile 52, 53 fluchten zueinander.

Figur 16 zeigt den Hebel 50 in einer verschwenkten Stellung, die Spannschraube 54 ist so verdreht, dass die Winkel 56, 57 aufeinander zu verlagert werden, was eine Verlagerung und Verschwenkung der beiden Hebelteile 52, 53 zueinander bewirkt. Der Abstand zwischen den Lagerungspunkten 160, 320 ist verringert, ebenso ist die Position des nicht dargestellten Lenkers relativ zu dem ebenfalls nicht darstellten Dämpfer variiert.

Figur 17 zeigt eine Variante der Einstellung, bei der statt einer Schraubenanordnung zueinander fluchtende, kreisförmig um die Schwenkachse 51 angeordnete Rastbohrungen 58 in beiden Hebelteilen 52, 53 angeordnet sind. Über ein Formschlusselement, beispielsweise einen Bolzen oder dergleichen, kann nach Ausrichtung der jeweiligen Rastbohrungen 58 zueinander eine formschlüssige Verriegelung in der gewünschten Winkelposition erreicht werden.

## Patentansprüche

1. Prothese mit einem Trägerteil (10)
- an dem Trägerteil (10) ist ein Schwenkgelenk (70) befestigt oder ausgebildet,
- über das Schwenkgelenk (70) ist eine distale Prothesenkomponente (20) an dem Trägerteil (10) befestigt,
- das Schwenkgelenk (70) ermöglicht eine Flexion und Extension der distalen Prothesenkomponente (20) relativ zu dem Trägerteil (10) um eine Gelenkachse (15),
- eine Dämpfereinrichtung (30), Federeinrichtung und/oder Aktuatoreneinrichtung ist zwischen der distalen Prothesenkomponente (20) und dem Trägerteil (10) angeordnet,
- die Dämpfereinrichtung (30), Federeinrichtung und/oder Aktuatoreneinrichtung ist mit einem ersten Lagerpunkt (310) an der distalen Prothesenkomponente (20) gelagert und mit einem zweiten Lagerpunkt (320) an einem Hebel (50) oder einem Lenker (40) gelagert,
- der Hebel (50) als Teil der Prothese ist schwenkbar um eine Schwenkachse (51) an der distalen Prothesenkomponente (20) gelagert,
- der Lenker (40) als Teil der Prothese ist mit einem distalen Abschnitt (42) an dem Hebel (50) und einem proximalen Abschnitt (41) an dem Trägerteil (10) gelagert,
**dadurch gekennzeichnet, dass** die Gelenkachse (15) durch das Trägerteil (10) hindurchgeht.

2. Prothese nach Anspruch 1, **dadurch gekennzeichnet, dass** an dem Trägerteil (10) ein Aufnahmeelement (60) zur Aufnahme eines Stumpfes ausgebildet oder befestigt ist.

3. Prothese nach Anspruch 1, **dadurch gekennzeichnet, dass** das Trägerteil (10) zur Aufnahme eines Stumpfes ausgebildet ist.

4. Prothese nach einem der voranstehenden Ansprüche, **dadurch gekennzeichnet, dass** der Hebel (50) als zweiarmiger Hebel (50) ausgebildet ist und die Schwenkachse (51) zwischen der Dämpfereinrichtung (30), Federeinrichtung und/oder Aktuatoreneinrichtung sowie dem Lenker (40) positioniert ist.

5. Prothese nach Anspruch 1, **dadurch gekennzeichnet, dass** der Hebel (50) als einarmiger Hebel (50) ausgebildet ist und der Lenker (40) und die Dämpfereinrichtung (30), Federeinrichtung und/oder Aktuatoreneinrichtung entfernt von der Schwenkachse (51) an dem Hebel (50) gelagert sind.

6. Prothese nach einem der voranstehenden Ansprüche, **dadurch gekennzeichnet, dass** der Lenker (40) und/oder die Dämpfereinrichtung (30), Federeinrichtung und/oder Aktuatoreneinrichtung verlagerbar an dem Hebel (50) gelagert sind.

7. Prothese nach einem der voranstehenden Ansprüche, **dadurch gekennzeichnet, dass** die Dämpfereinrichtung (30), Federeinrichtung und/oder Aktuatoreneinrichtung, der Lenker (40) und/oder der Hebel (50) längenveränderlich ausgebildet sind.

8. Prothese nach einem der voranstehenden Ansprüche, **dadurch gekennzeichnet, dass** die distale Prothesenkomponente (20) einen Hohlraum (25) ausbildet, in dem der Hebel (50) und die Dämpfereinrichtung (30), Federeinrichtung und/oder Aktuatoreneinrichtung angeordnet sind.

9. Prothese nach Anspruch 8, **dadurch gekennzeichnet, dass** der Lenker (40) den Zugang zu dem Hohlraum (25) zumindest teilweise abdeckt.

10. Prothese nach Anspruch 8, **dadurch gekennzeichnet, dass** der Lenker (40) die Kontur der distalen Prothesenkomponente (20) ergänzt, insbesondere als Wadenabschnitt ausgebildet ist.

11. Prothese nach einem der voranstehenden Ansprüche, **dadurch gekennzeichnet, dass** die distale Prothesenkomponente (20) unmittelbar an dem Trägerteil (10) oder Aufnahmeelement (60) an zumindest einem Lagerpunkt (71, 72) gelagert ist und der zumindest eine Lagerpunkt (71, 72) medial oder lateral an dem Trägerteil (10) oder dem Aufnahmeelement (60) positioniert ist.

12. Prothese nach einem der voranstehenden Ansprüche, **dadurch gekennzeichnet, dass** die Gelenkachse (15) im Bereich einer natürlichen Gelenkachse einer Gliedmaße positioniert ist.

13. Prothese nach einem der voranstehenden Ansprüche, **dadurch gekennzeichnet, dass** der Lenker (40) verstellbar an dem Trägerteil (10) oder dem Aufnahmeelement (60) gelagert ist.

14. Prothese nach einem der voranstehenden Ansprüche, **dadurch gekennzeichnet, dass** die Gelenkachse (15) proximal zu einem distalen Ende des Trägerteils (10) oder Aufnahmeelementes (60) positioniert ist.

15. Prothese nach einem der voranstehenden Ansprüche, **dadurch gekennzeichnet, dass** Lagerpunkte (310, 510; 320) an der distalen Prothesenkomponente (20) und/oder dem Hebel (50) verstellbar ausgebildet sind.

16. Prothese nach einem der voranstehenden Ansprüche, **dadurch gekennzeichnet, dass** sie eine Sperre zur Sperrung der Flexion und/oder Extension ausweist.

## Claims

1. A prosthesis comprising a support part (10)
- a swivel joint (70) is fastened or formed on the support part (10),
- a distal prosthesis component (20) is fastened on the support part (10) via the swivel joint (70),
- the swivel joint (70) allows for a flexion and extension of the distal prosthesis component (20) about an axis of the joint (15) relative to the support part (10),
- a damping device (30), spring device and/or actuator device is situated between the distal prosthesis component (20) and the support part (10),
- the damping device (30), spring device and/or actuator device is mounted by means of a first bearing point (310) on the distal prosthesis component (20) and by means of a second bearing point (320) on a lever (50) or a connecting rod (40),
- the lever (50) as part of the prosthesis is mounted on the distal prosthesis component (20) so as to swivel about a swivel axis (51),
- the connecting rod (40) as part of the prosthesis is mounted on the lever (50) via a distal portion (42) and is mounted on the support part (10) via a proximal portion (41),
**characterized in that** the axis of the joint (15) extends through the support part (10).

2. The prosthesis as claimed in claim 1, **characterized in that** a receiving element (60) for receiving a stump is formed on or fastened to the support part (10).

3. The prosthesis as claimed in claim 1, **characterized in that** the support part (10) is designed for receiving a stump.

4. The prosthesis as claimed in one of the preceding claims, **characterized in that** the lever (50) is designed as a double-armed lever (50) and the swivel axis (51) is positioned between the damping device (30), spring device and/or actuator device and the connecting rod (40).

5. The prosthesis as claimed in claim 1, **characterized in that** the lever (50) is designed as a single-armed lever (50), and the connecting rod (40) and the damping device (30), spring device and/or actuator device are mounted on the lever (50) at a distance from the swivel axis (51).

6. The prosthesis as claimed in one of the preceding claims, **characterized in that** the connecting rod (40) and/or the damping device (30), spring device and/or actuator device are displaceably mounted on the lever (50) .

7. The prosthesis as claimed in one of the preceding claims, **characterized in that** the damping device (30), spring device and/or actuator device, the connecting rod (40) and/or the lever (50) are designed to be variable in length.

8. The prosthesis as claimed in one of the preceding claims, **characterized in that** the distal prosthesis component (20) forms a hollow space (25) in which the lever (50) and the damping device (30), spring device and/or actuator device are situated.

9. The prosthesis as claimed in claim 8, **characterized in that** the connecting rod (40) at least partially covers the access to the hollow space (25).

10. The prosthesis as claimed in claim 8, **characterized in that** the connecting rod (40) supplements the contour of the distal prosthesis component (20), in particular as a calf portion.

11. The prosthesis as claimed in one of the preceding claims, **characterized in that** the distal prosthesis component (20) is mounted directly on the support part (10) or receiving element (60) at at least one bearing point (71, 72), and the at least one bearing point (71, 72) is positioned medially or laterally on the support part (10) or the receiving element (60).

12. The prosthesis as claimed in one of the preceding claims, **characterized in that** the axis of the joint (15) is positioned in the region of a natural axis of the joint of a limb.

13. The prosthesis as claimed in one of the preceding claims, **characterized in that** the connecting rod (40) is displaceably mounted on the support part (10) or the receiving element (60).

14. The prosthesis as claimed in one of the preceding claims, **characterized in that** the axis of the joint (15) is positioned proximally to a distal end of the support part (10) or the receiving element (60).

15. The prosthesis as claimed in one of the preceding claims, **characterized in that** bearing points (310, 510; 320) are displaceably formed on the distal prosthesis component (20) and/or the lever (50).

16. The prosthesis as claimed in one of the preceding claims, **characterized in that** the prosthesis comprises a blocking device for blocking the flexion and/or extension.

## Revendications

1. Prothèse comprenant une partie de support (10), dans laquelle
- une articulation pivotante (70) est fixée ou réalisée sur la partie de support (10),
- un composant de prothèse distal (20) est fixé à la partie de support (10) par l'intermédiaire de l'articulation pivotante (70),
- l'articulation pivotante (70) permet une flexion et une extension du composant de prothèse distal (20) par rapport à la partie de support (10) autour d'un axe d'articulation (15),
- un dispositif amortisseur (30), un dispositif à ressort et/ou un dispositif actionneur est disposé entre le composant de prothèse distal (20) et la partie de support (10),
- le dispositif amortisseur (30), le dispositif à ressort et/ou le dispositif actionneur est monté par un premier point d'appui (310) sur le composant de prothèse distal (20) et par un deuxième point d'appui (320) sur un levier (50) ou sur une bielle (40),
- le levier (50), en tant que partie de la prothèse, est monté sur le composant de prothèse distal (20) de manière à pouvoir pivoter autour d'un axe de pivotement (51),
- la bielle (40), en tant que partie de la prothèse, est montée avec une portion distale (42) sur le levier (50) et avec une portion proximale (41) sur la partie de support (10),
**caractérisée en ce que**
l'axe d'articulation (15) traverse la partie de support (10).

2. Prothèse selon la revendication 1,
**caractérisée en ce qu'**un élément de réception (60) destiné à recevoir un moignon est réalisé ou fixé sur la partie de support (10).

3. Prothèse selon la revendication 1,
**caractérisée en ce que** la partie de support (10) est conçue pour recevoir un moignon.

4. Prothèse selon l'une des revendications précédentes,
**caractérisée en ce que** le levier (50) est conçu comme un levier à deux bras (50), et l'axe de pivotement (51) est positionné entre le dispositif amortisseur (30), le dispositif à ressort et/ou le dispositif actionneur ainsi que la bielle (40).

5. Prothèse selon la revendication 1,
**caractérisée en ce que** le levier (50) est conçu comme un levier à un seul bras (50), et la bielle (40) et le dispositif amortisseur (30), le dispositif à ressort et/ou le dispositif actionneur sont montés sur le levier (50) à distance de l'axe de pivotement (51).

6. Prothèse selon l'une des revendications précédentes,
**caractérisée en ce que** la bielle (40) et/ou le dispositif amortisseur (30), le dispositif à ressort et/ou le dispositif actionneur sont montés sur le levier (50) de manière à pouvoir être déplacés.

7. Prothèse selon l'une des revendications précédentes,
**caractérisée en ce que** le dispositif amortisseur (30), le dispositif à ressort et/ou le dispositif actionneur, la bielle (40) et/ou le levier (50) sont de longueur variable.

8. Prothèse selon l'une des revendications précédentes,
**caractérisée en ce que** le composant de prothèse distal (20) forme une cavité (25) dans laquelle sont disposés le levier (50) et le dispositif amortisseur (30), le dispositif à ressort et/ou le dispositif actionneur.

9. Prothèse selon la revendication 8,
**caractérisée en ce que** la bielle (40) recouvre au moins partiellement l'accès à la cavité (25).

10. Prothèse selon la revendication 8,
**caractérisée en ce que** la bielle (40) vient compléter le contour du composant de prothèse distal (20), en particulier est conçue comme une portion pour mollet.

11. Prothèse selon l'une des revendications précédentes,
**caractérisée en ce que** le composant de prothèse distal (20) est monté directement sur la partie de support (10) ou sur l'élément de réception (60) en au moins un point d'appui (71, 72), et ledit au moins un point d'appui (71, 72) est positionné en direction médiale ou latérale sur la partie de support (10) ou sur l'élément de réception (60).

12. Prothèse selon l'une des revendications précédentes,
**caractérisée en ce que** l'axe d'articulation (15) est positionné dans la zone d'un axe d'articulation naturel d'un membre.

13. Prothèse selon l'une des revendications précédentes,
**caractérisée en ce que** la bielle (40) est montée de manière réglable sur la partie de support (10) ou sur l'élément de réception (60).

14. Prothèse selon l'une des revendications précédentes,
**caractérisée en ce que** l'axe d'articulation (15) est positionné en direction proximale par rapport à une extrémité distale de la partie de support (10) ou de l'élément de réception (60).

15. Prothèse selon l'une des revendications précédentes,
**caractérisée en ce que** des points d'appui (310, 510 ; 320) sur le composant de prothèse distal (20) et/ou sur le levier (50) sont conçus de manière réglable.

16. Prothèse selon l'une des revendications précédentes,
**caractérisée en ce qu'**elle présente un blocage pour bloquer la flexion et/ou l'extension.
